# EUROPÄISCHE PATENTSCHRIFT

(11) **EP 4 313 236 B1**
(45) Veröffentlichungstag und Bekanntmachung des Hinweises auf die Patenterteilung: **23.09.2026**
(21) Anmeldenummer: 22714348.4
(22) Anmeldetag: 16.03.2022
(51) Int. Cl.: A61M 25/00, A61M 39/06, A61M 39/22, A61M 39/26, A61M 39/28

(54) **KATHETERVENTIL ZUM STEUERN DES FLUIDFLUSSES EINES MEDIUMS**
CATHETER VALVE FOR CONTROLLING THE FLUID FLOW OF A MEDIUM
VALVE DE CATHÉTER POUR RÉGULATION D'ÉCOULEMENT D'UN FLUIDE

(30) Priorität: 25.03.2021 DE 102021001563
(43) Veröffentlichungstag der Anmeldung: 07.02.2024
(73) Patentinhaber: UROMED KURT DREWS KG, 22113 Oststeinbek (DE)
(72) Erfinder: SCHWARZ, Werner, 83324 Ruhpolding (DE)
(74) Vertreter: Eisenführ Speiser
(86) Internationale Anmeldenummer: PCT/DE2022/000026
(87) Internationale Veröffentlichungsnummer: WO 2022/199734

(56) Entgegenhaltungen:
- EP-A1- 3 679 978
- US-A- 4 243 034
- US-A1- 2018 245 699

## Beschreibung

Die Erfindung betrifft ein Katheterventil zum Steuern des Fluidflusses eines Mediums, insbesondere ein selbstschließendes Katheterventil, wobei ein Grundkörper einen Ein- sowie einen Ausgang, einen flexiblen Tubus und als Deckel einen in Längsrichtung beweglichen Schieber sowie am Eingang des Katheterventils eine stufenförmige Rasterung zum Einbinden einer Zuleitung aufweist.

Harninkontinenz ist ein häufig anzutreffender Zustand, der aus einer Vielzahl von Gründen auftreten kann, beispielsweise Krankheit, Verletzungen oder Entkräftung. Ein Harnkontinenzzustand ist üblicherweise durch die Schwäche oder das totale Fehlen der Funktion von denjenigen Muskeln charakterisiert, die die Expansion und Kontraktion des Harnröhrenschließmuskels steuern. Bis heute sind keine operativen Verfahren bekannt, die in zufriedenstellender Weise einen Inkontinenzzustand derart korrigieren, dass die Blasensteuerung im Wesentlichen wiederhergestellt wird.

Versuche, den Inkontinenzzustand zu verbessern, umfassen die Verwendung von passiven Vorrichtungen, wie beispielsweise von einem Sammelbeutel, der vom Benutzer getragen wird, um den vom Harnleiter abgegebenen Urin zu sammeln. Ein Nachteil dieser Lösung besteht darin, dass sich die Blase kontinuierlich in den Beutel entleert, ohne dass hierbei der Benutzer irgendeine Steuerungsmöglichkeit der Urinabgabe besitzt. Ein weiterer Nachteil dieser Lösung ist darin zu sehen, dass der Sammelbeutel den Benutzer begleiten muss und somit dessen Aktivitäten behindert.

Die in der Urologie angewendeten Katheterventile werden immer häufiger für chronische Patienten eingesetzt. Insbesondere in der Geriatrie müssen Patienten Katheterventile über Jahre hinweg verwenden. Diesem Umstand wurde bis heute kaum Rechnung getragen.

Um diese Bedingungen zu erfüllen, müssen sowohl formgeberische Aspekte berücksichtigt werden, wie auch konstruktive Aspekte, die von der Formgebung teilweise diktiert werden.

Versuche zur Lösung von derartigen Problemen, wie beispielsweise dem Fehlen einer Steuerungsmöglichkeit, dem großen Volumen, der Unbequemlichkeit und der Verhinderung von Aktivitäten, haben zur Entwicklung von mit Ventilen versehenen Inkontinenzsteuervorrichtungen geführt, durch die äußere Sammelsysteme entfallen können und die es ermöglichen, dass der Benutzer die Urinentleerung aus der Harnröhre manuell steuern kann. Beispiele derartiger Vorrichtungen sind der US-PS 35 03 400, US-PS 39 39 821 und der US-PS 40 24 855 beschrieben.

Bekannt ist ebenfalls die US-PS 37 31 670, bei der ein bistabiles magnetisches Ventilelement eine erste magnetische Betätigung von der geschlossenen Stellung zurück in eine offene Stellung erfordert.

Obwohl eine derartige Vorrichtung dem Benutzer eine gewisse Entleerungsteuerung ermöglicht, ist sie nicht ausreichend betriebssicher. Wenn das Ventil beispielweise einer ersten Betätigung in eine offene Stellung unterzogen wird und die Entleerung aufhört, kann der Benutzer vergessen, eine zweite Betätigung durchzuführen, und das Ventil in eine geschlossene Position zurückzuführen, während es sich noch in der geöffneten Stellung befindet. Wenn das Ventil in unbeabsichtigter Weise in der offenen Stellung geblieben ist, kann es zu einer unerwarteten Entleerung kommen.

Die Gefahr eines unbeabsichtigten Öffnens verbleibt auch bei einem Katheterventil, wie es in der WO 93/24173 beschrieben ist. Hierbei verschließt im Normalzustand ein Federelement den Durchfluss durch einen flexiblen Leitungsabschnitt. Mit Hilfe eines Betätigungselementes in Form einer Taste, die gegen die Kraft des Federelementes zu betätigen ist, kann der Verschluss aufgehoben und das Ventil geöffnet werden. Auch bei dieser Anordnung kann das Betätigungsorgan relativ leicht einem Druck ausgesetzt werden, der durch ein Anstoßen oder eine zufällige Berührung ausgeübt wird, so dass auch dieses Ventil dahingehend nachteilig ist, dass ein ungewolltes Öffnen nicht ausgeschlossen werden kann.

Ferner sind Katheterventile bekannt, die den flexiblen Leitungsabschnitt zum Verschließen nicht zusammendrücken, sondern abknicken. Die EP 0 088 871 A1 funktioniert nach diesem Prinzip. Hierbei ist ein Abschnitt der flexiblen Leitung an einer Hülse befestigt, so dass ein Verschieben der Hülse relativ zu einem Gehäuse, in dem ein anderer Abschnitt der flexiblen Leitung befestigt ist, zu einer Verkürzung der flexiblen Leitung und zu einer daraus resultierenden Knickung der Leitung führt, indem ein Teil der Leitung seitlich ausweicht. Durch eine Feder wird die Hülse in einer Stellung gehalten, in der die Leitung wie beschrieben geknickt ist, und der Durchfluss durch die Leitung somit verschlossen ist.

Die mehrfache Knickung der Leitung, die als Schlauch ausgebildet ist, führt jedoch zu neuen Problemen. Die fortwährende knickende Verformung des Schlauches führt zu Beschädigungen der Schlauchwandung und macht eine häufige Kontrolle des Ventils und unter Umständen sogar ein häufiges Austauschen des Ventils erforderlich. Außerdem ist auch bei der beschriebenen Ausführung nicht gewährleistet, dass ein unbeabsichtigtes Verschieben der Hülse und damit ein Öffnen des Durchflusses ausreichend verhindert wird. Ferner ist ein derartiges Katheterventil dahingehend verbesserungswürdig, dass seine Konstruktion unnötig kompliziert ist.

Aus der US-PS 21 97 995 ist ein sogenanntes Quetschventil bekannt, bei dem in einer dargestellten Ausführungsform zwei voneinander unabhängig betätigbare Absperrelemente auf einen flexiblen Leitungsabschnitt zur Steuerung des Durchflusses einwirken. Jedes der Absperrelemente ist einzeln beweglich. Bei dieser Ausführungsform sind die Absperrelemente einander gegenüberliegend angeordnet und die Einwirkung mittels der Ansperrelemente auf den flexiblen Leitungsabschnitt erfolgt an einander gegenüberliegenden Stellen des flexiblen Leitungsabschnitts.

Weiterhin ist aus der EP 3 247 450 B1 ein Katheterventil zur Steuerung eines Fluidflusses bekannt, welches über ein bewegliches Verschlusselement verfügt, das verschiedene Stellungen einnehmen kann. Das Verschlusselement ist dabei durch einen magnetisch wirkenden Ventilantrieb antreibbar.

Die Nachteile aller vorgenannten Lösungen sind:
- Komplizierter konstruktiver Aufbau
- teure Herstellung
- komplizierte Bedienbarkeit
- schwierige Reinigung und Sterilisierung
- viele Bauteile notwendig
- Magnetkörper/-kraft steuert indirekt Verschlusselemente
- Keine (Ein-)Handbedienung möglich
- Keine Dauerstellung/Öffnung möglich

Aus der DE 10 2019 100 509 B4 ist ein Katheterventil zur Einstellung einer Durchflussmenge eines mit einem Katheter austauschbaren Mediums bekannt, welches eine Ventilgehäusestruktur umfasst, die einen Katheterventilinnenraum des Katheterventils bereichsweise begrenzt und eine Bodenwand, die zur Auflage des Katheterventils ausgebildet ist, eine der Bodenwand gegenüberliegend angeordnete Oberwand sowie wenigstens eine, die Bodenwand und die Oberwand verbindende Seitenwand besitzt. Das Katheterventil umfasst zudem ein Leitungselement zum Leiten des Mediums, welches sich in Längserstreckungsrichtung der Ventilgehäusestruktur durch den Katheterventilinnenraum erstreckt, eine Verschlussvorrichtung zum Sperren und Freigeben eines Durchflusses des Mediums durch das Leitungselement, ein Stellelement, welches relativ zu der Ventilgehäusestruktur beweglich gelagert ist und mittels welchem die Verschlussvorrichtung zwischen einer Sperrstellung und einer Freigabestellung verstellbar ist. Das Stellelement ist verschiebbar an oder in der Oberwand angeordnet. Ein weiterer Aspekt der Erfindung betrifft eine Verschlussvorrichtung für das Katheterventil.

Diese Lösung weist folgende Nachteile auf:
- mögliche Materialermüdung der Federarmelemente und des als O-Ringes ausgebildetem Federelements bei mehrmaliger Betätigung kann zur Undichtigkeit des Katheterventils führen,
- hoher Kraftaufwand zum Halten der Öffnung erforderlich,
- keine vollständiges Öffnungsvolumina des Ventilschlauchs, bedingt durch die konstruktive Bauweise, führt für zu einer geringen Durchflussmenge und dadurch zu langer Entleerungszeit,
- komplexe Bedienbarkeit, eine Dauerstellung/Öffnung des Ventils muss durch eine umständliche Betätigung des Schiebers aktiviert werden,
- Gefahr, dass beim Entfernen des Urinbeutels vergessen wird, die Dauerstellung am Ventil zu deaktivieren, dadurch unbeabsichtigter Urinausfluss möglich,
- großes Bauvolumen bedingt durch lange Federarmelemente beeinträchtigt den Tragekomfort für den Patienten,
- besteht aus vielen Einzelbauteilen,
- geringer Abstand von Betätigungselement zum Ausgang des Ventils erhöht die Gefahr, dass dem Patienten Urin über die Hand oder Finger fließen kann (Hygiene),
- eine unbeabsichtige Dekonnektierung oder Lösen des Katheters ist möglich.

Die US 4 243 034 beschreibt ein gattungsgemäßes Katheterventil zum Steuern des Fluidflusses eines Mediums. Weitere Katheterventile sind aus der EP 3 679 978 A1 und der US 2018/245699 A1 bekannt.

Schließlich ist aus der DE 29 41 278 A1 ein Kanülen- oder Katheteraggregat bekannt, welches ein Schlauchstück und einen beweglichen Druckkörper aufweist, wobei ein an der Außenseite der den Kanal umgebenden Wand gelagerter Schieber in Längsrichtung des Kanals hin und her verschiebbar ist und eine dem Druckkörper zugewandte Schrägfläche aufweist, die bei Verschiebung des Schiebers den Druckkörper zwischen den beiden Lagern umstellt.

Diese Lösung weist folgende Nachteile auf:
- keine selbstschließende Funktion vorgesehen, da der Schieber zum Schließen des Ventils betätigt werden muss.
- zum Öffnen des Ventils muss der leicht biegsame Schlauch die Druckkörper auseinander drücken. Das bedeutet, dass das Schlauchmaterial eine bestimmte Steifigkeit besitzen muss. Dies kann bei dauerhafter Anwendung zu einer Materialermüdung führen und erhöht dadurch das Risiko einer Fehlfunktion. Außerdem kann ein steiferes Material zu Lasten der Dichtigkeit führen.
- es wird radial um eine Kanüle abgedichtet und nicht der flexible Schlauch komplett zusammengedrückt. Die Druckkörper sind kugelförmig ausgelegt und würden daher beim zusammendrücken ohne Kanüle keine sichere Dichtigkeit am Schlauch erreichen.

Die DE 10 2013 012 158 A1 beschreibt ein Quetschventil, das über ein schlauchförmiges Ventilglied verfügt, das von einem ringförmigen Betätigungsglied umschlossen ist und das an entgegengesetzten Seiten von Quetschelementen flankiert ist. Die Quetschelemente sind durch eine Betätigungsbewegung des Betätigungsgliedes relativ zu dem Ventilglied beweglich und geben wahlweise einen Ventilkanal des Ventilgliedes entweder frei oder sperren es ab.

Nachteile dieser Lösung sind:
- die Quetschelemente bewegen sich in einen bestimmten Winkel in axialer Längsrichtung auf den flexiblen Schlauch zu. Das kann bei häufiger Verwendung des Ventils am Schlauch zu einem Materialabrieb führen und eine sichere Dichtigkeit gefährden.
- zusätzlich wirkt eine axiale längs gerichteter Kraft auf den lose liegenden Schlauch, das zu einer Verschiebung der Position des Schlauches führen kann. Dadurch kann es zu Funktionsstörungen oder einer Undichtigkeit des Ventils kommen.
- zum Öffnen und Halten der Position muss gegen eine Federkraft geschoben werden, das heißt der Anwender muss beim Entleeren der Blase eine relativ hohe Kraft aufwenden.
- Die Feder ist ein weiteres Bauteil und führt zu mehr Material- und Montagekosten.

Letztendlich ist aus der DE 10 2007 002 765 B3 ein weiteres Quetschventil bekannt, welches in der Ausgestaltungsform eine Kombination der vorherigen beiden genannten DE 29 41 278 A1 und DE 10 2013 012 158 A1 bekannt.. Die Nachteile dieser Lösung sind im Wesentlichen die gleichen, wie oben bereits genannt.

Der Erfindung liegt die Aufgabe zugrunde, ein Katheterventil zu entwickeln, welches:
1. ergonomisch gut geformt ist, so dass der Patient auch wenn er lange Zeit bettlägerig ist, sich daran gewöhnen kann,
2. da bei Langzeitpatienten die Pflegekosten besonders wesentlich sind, muss das Katheterventil äußerst preiswert sein,
3. da Geriatrische Patienten in ihrer Motorik oftmals reduziert sind, bedingt dies, dass das Katheterventil äußerst einfach und mit einer Hand bedient werden muss,
4. da oftmals eine Langzeitverwendung erfolgt, setzt dies voraus, dass das gesamte Ventil ohne schwierige Demontage durchspülbar und sterilisiert werden kann,
5. ein unbeabsichtigtes Lösen des Katheters vom Katheterventil muss verhindert werden, wodurch die Gefahr einer Infektion vermieden wird.

Die Aufgabe wird gelöst durch ein Katheterventil mit den Merkmalen des Anspruchs 1 und durch ein Katheterventil mit den Merkmalen des Anspruchs 2. Vorteilhafte Ausgestaltungen sind in den Unteransprüchen angegeben.

Die Lösung der Aufgabe wird erfindungsgemäß dadurch erreicht, dass der zwei gegenläufige Ablaufschrägen aufweisende Grundkörper (1) zwei schräge gegenläufige Kammern (12a; 13a) für die Betätigung und Führung von zwei vertikal beweglichen Magneten (10; 11) aufweist, die an zwei gegenüberliegenden Seiten des Ventilschlauches (6) angeordnet sind und dessen Durchfluss wahlweise verschließen oder öffnen, wobei am Ausgang (3) des Katheterventils ein Adapter (17) einzuführen und zu befestigen ist, der - neben dem längsbeweglichen Schieber (7) - zu einer geöffneten Dauerstellung des Katheterventils führt. Das Aufschieben des Adapters (17) führt zu einer Längsverschiebung des Schiebers (7) hin in dessen Öffnungsstellung.

In einer weiteren Ausführungsform der erfindungsgemäßen Lösung weist das Katheterventil im Inneren des zwei seitlich gegenüberliegende vertikale Kammern (12b; 13b) aufweisenden Grundkörpers (1) am längsbeweglichen Schieber (7) eine Ablaufschräge (8) sowie eine schräge Kammer (12a) für die Betätigung und Führung von einem vertikal beweglichen Magneten (10) aufweist, der an einer Seite des Ventilschlauches (6) angeordnet ist und dessen Durchfluss wahlweise verschließt oder öffnet, wobei am Ausgang des Katheterventils ein Adapter (17) einzuführen und zu befestigen ist, der - neben dem längsbeweglichen Schieber (7) - zu einer geöffneten Dauerstellung des Katheterventils führt. Das Aufschieben des Adapters (17) führt auch hier zu einer Längsverschiebung des Schiebers (7) hin in dessen Öffnungsstellung.

An der stufenförmigen Rasterung des Eingangs des Katheterventils ist eine integrierte oder beidseitig aufsteckbare Sicherung angeordnet.

Die Sicherung ist dabei als U-förmig ausgebildete Doppelseiten-Klemme ausgebildet.

Der Adapter weist vorzugsweise einen Überwurfring auf, der auf einer Rasterung des Ausgangs des Katheterventils über- oder eingreift oder ist so ausgebildet, dass er flexibel und fest übergestülpt auf dem Ausgang des Katheterventils sitzt.

Das Katheterventil kann erfindungsgemäß auch so aufgebaut sein, dass anstelle des längsbeweglichen Schiebers ein einseitig oder beidseitig angeordneter Druckknopf zur Betätigung der beiden Magneten eingebaut ist.

Ein Entfernen des Adapters aus der Aufnahme des Katheterventils führt zu einem automatischen Schließen der Magneten und damit des Tubus. Bevorzugt wird dieses Schließen ausschließlich durch die Magnetkraft bewirkt, die die rückstellende Kraft bereitstellt. Optional, aber weniger bevorzugt, könnte dieses Schließen durch eine zusätzliche Federkraft unterstützt werden.

Das Katheterventil dient als Schnittstelle zwischen dem im Patienten liegenden Katheter oder dem Kathetertrichter und dem Urinbeutel. Es kann manuell zum Wasserlassen geöffnet werden, um den Urinfluss herzustellen. Für einen durchgehenden Urinfluss kann es nachts auf Dauerstellung gesetzt werden. Es ist für Langzeitanwendung (>30 Tage) sowie zur Durchführung von Blasentraining geeignet.

Besondere Vorteile der Erfindung:
- automatisches Öffnen und Dauerstellung des Durchflusses durch Einführen des Adapters,
- automatisches Schließen des Ventils durch Entfernung des Adapters, dadurch keine zusätzliche Betätigung notwendig,
- ergonomisch vorteilhafte Formgebung und damit sehr leichte Bedienung auch durch bettlägerige Patienten,
- preiswerte Herstellung durch geringe Anzahl von Einzelteilen und einfache Montage,
- einfache Reinigung und Sterilisierung möglich,
- geringer Kraftaufwand zum Halten des Schiebers in der Öffnungsstellung,
- vollständiges Öffnungsvolumen für schnelle Urinentleerung,
- geringe Baugröße für besseren Tragekomfort,
- Sicherungs-Doppelseiten-Klemme gegen unbeabsichtige Dekonnektierung von Katheter und Katheterventil,
- kurzer Öffnungsweg des Schiebers für einfache Bedienbarkeit.

Eine Besonderheit ist der spezielle Aufbau des Katheterventils durch das erfindungsgemäße Zusammenwirken des sehr innovativ aufgebauten, längsverschieblichen Schiebers mit seinen integrierten beiden Ablaufschrägen und den integrierten beiden schrägen Kammern sowie den beiden vertikalen Kammern des Grundkörpers zum vertikalen Bewegen der beiden Magnete, die den Ventilschlauch schließen oder öffnen.

Wie bereits beschrieben ist eine erfindungsmäßige Ausführung auch mit einem Magneten möglich. In diesem Fall weist der Grundkörper an seinem Boden auf der Höhe des Magneten als Gegenstück eine metallische Platte mit magnetischen Eigenschaften auf.

Die beiden vertikalen Kammern begrenzen dabei eine vertikale Führung, in der die beiden Magneten oder der eine Magnet beweglich geführt ist. Längsverschiebung des Schiebers führt dazu, dass der Magnet oder die Magnete an den Ablaufschrägen entlanggeführt und voneinander wegbewegt werden, während sie in der vertikalen Führung geführt sind. Bei Entfernen des Adapters führt die magnetische Anziehungskraft dazu, dass das Katheterventil in den Ausgangszustand zurückkehren möchte. Ist diese Magnetkraft ausreichend groß, so geht das Katheterventil selbsttätig wieder in den geschlossenen Zustand über, sobald der Adapter (17) wieder abgezogen wird.

### Ausführungsbeispiel

Anhand eines Ausführungsbeispiels soll nachfolgend die Erfindung näher erläutert werden.

Dabei zeigt die
- Figur 1 - das Katheterventil in isometrischer Ansicht mit geschlossenen Magneten und Doppelseiten-Klemme
- Figur 2 - das Katheterventil in isometrischer Ansicht und den Grundkörper in Schnittdarstellung mit geschlossenen Magneten
- Figur 3 - das Katheterventil im Querschnitt mit geschlossenen Magneten
- Figur 4 - das Katheterventil in isometrischer Ansicht und geöffneten Magneten
- Figur 5 - das Katheterventil im Querschnitt mit geöffneten Magneten
- Figur 6 - das Katheterventil in isometrischer Ansicht und mit abgehobenem Schieber, Doppelseiten-Klemme und Ventilschlauch
- Figur 7 - das Katheterventil im geschlossenen Zustand
- Figur 8 - das Katheterventil im geöffneten Zustand
- Figur 9 - die Magneten geschlossen
- Figur 10 - die Magneten geöffnet
- Figur 11 - das Katheterventil mit Doppelseiten-Klemme, Adapter und Katheter in der Seitenansicht
- Figur 12 - das Katheterventil im Schnitt mit Doppelseiten-Klemme, Adapter und Katheter in der Seitenansicht
- Figur 13 - das Katheterventil mit Katheter und aufgestecktem Adapter in der Seitenansicht
- Figur 14 - das Katheterventil mit aufgestecktem Adapter im Schnitt und Katheter
- Figur 15 - Doppelseiten-Klemme in isometrischer Ansicht
- Figur 16 - Doppelseiten-Klemme in der Frontansicht mit Spitzformgeometrie
- Figur 17 - Doppelseiten-Klemme in der Seitenansicht
- Figur 18 - Katheterventil mit aufsteckbarer Kathetersicherung
- Figur 19 - Katheterventil mit integrierter Kathetersicherung

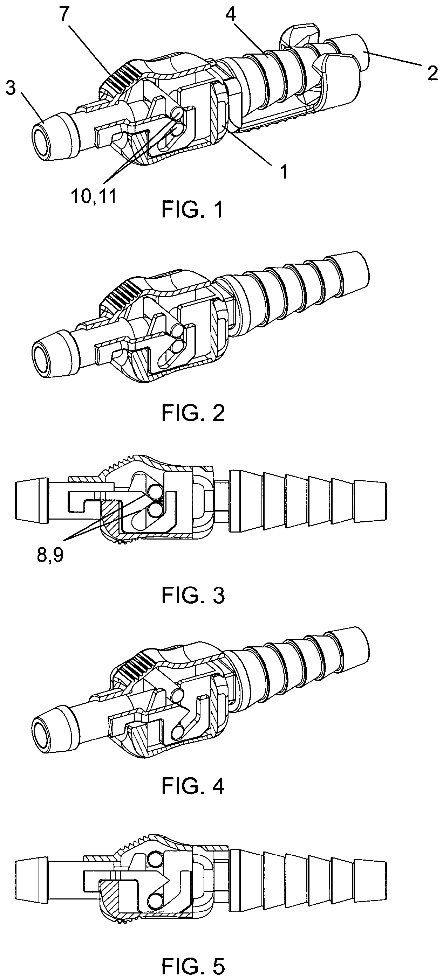

Das Katheterventil weist einen Grundkörper 1 mit einem Eingang 2 und einem Ausgang 3 auf (Figur 1, 2, 3, 4, 5, 6, 7, 8, 9, 10). Am Eingang 2 befindet sich eine stufenförmige Rasterung 4 zur Aufnahme des Katheters 5 vom Patienten. Durch den Grundkörper 1 ist der Ventilschlauch 6 geführt. Auf dem Grundkörper 1 ist ein längsbeweglicher Schieber 7 angeordnet, an dem sich im Inneren des Grundkörpers 1 zwei gegenläufig angeordnete Ablaufschrägen 8; 9 befinden. Durch eine Längsbewegung des Schiebers 7 werden die beiden Magnete 10; 11 über die Ablaufschrägen 8; 9 in vertikaler Richtung auf oder abwärts bewegt und schließen damit den Querschnitt des Ventilschlauches 6 oder öffnen ihn. Die beiden Magnete 10;11 bewegen sich dabei in zwei schräg angeordneten Kammern 12a; 13a gegen zwei vertikal laufenden Kammern 12b; 13b nach oben oder nach unten.

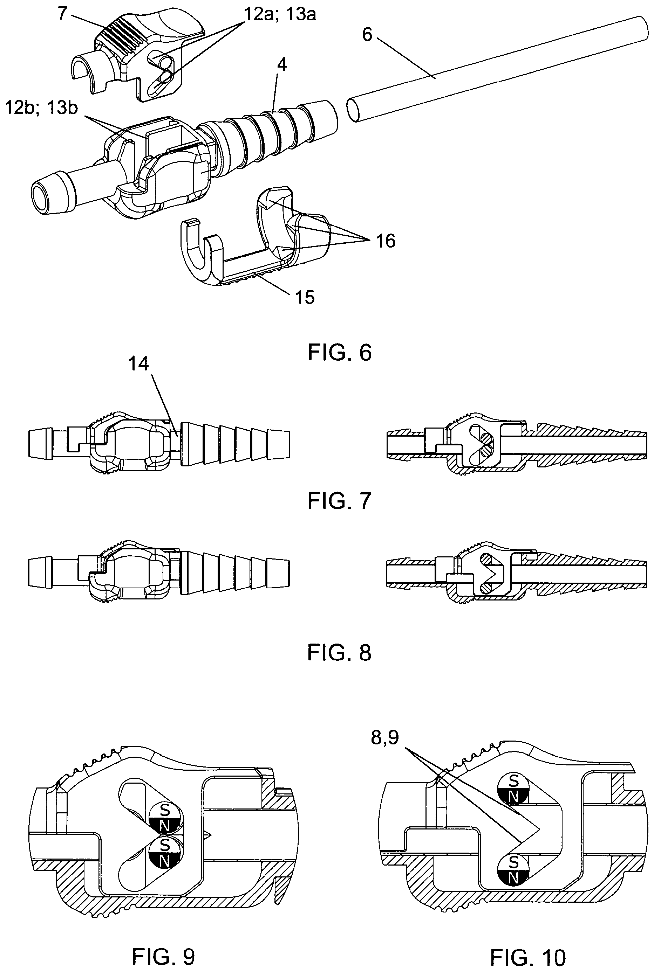

An der stufenförmigen Rasterung 4 ist zum Grundkörper 1 eine Nut 14 angeordnet, in die eine Seite der Doppelseiten-Klemme 15 eingreift (Figur 7). Die andere Seite der Doppelseiten-Klemme 15 greift auf die stufenförmige Rasterung 4 und den Katheter 5 zu, der auf die stufenförmige Rasterung 4 aufgeschoben wird.

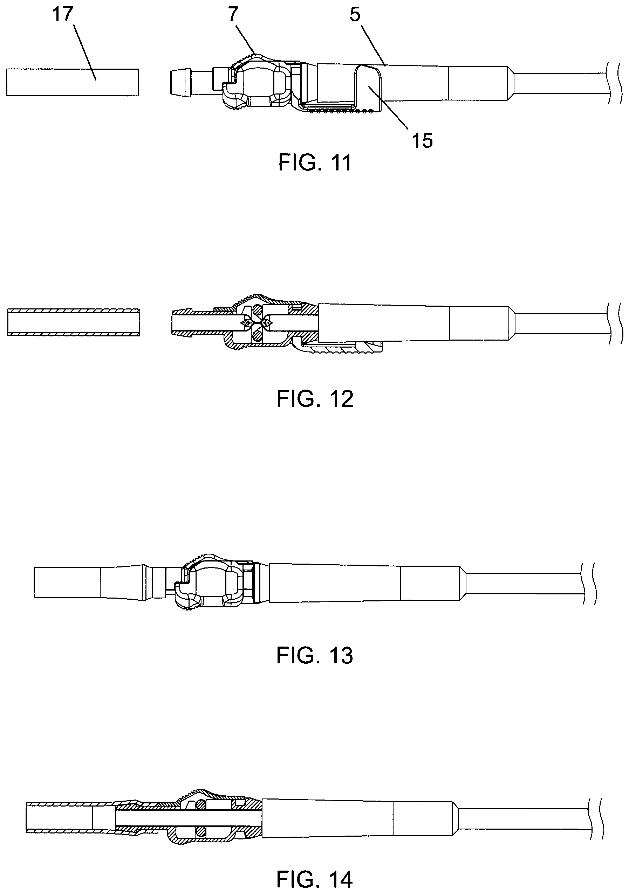

Dabei weist die Doppelseiten-Klemme 15 auf der zum Katheter 5 ausgerichteten Seite eine spezielle Spitzformgeometrie 16 mit vorzugsweise drei Spitzen auf, wodurch erreicht wird, dass die drei Spitzen bei Zugbelastung in das Silikonmaterial des Katheters 5 eingreifen und verhindern so eine unbeabsichtigte Dekonnektierung (Figur 11, 12, 15, 16, 17, 18).

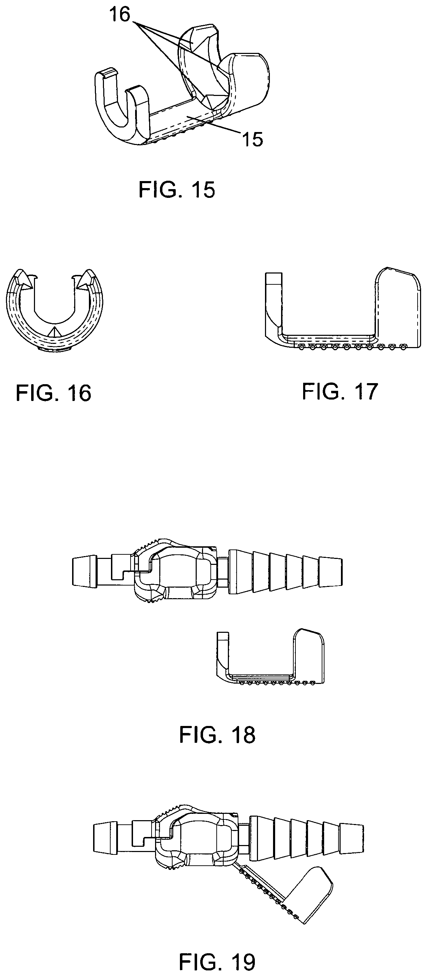

(Figur 19) zeigt eine integrierte Ausführungsform, in der die Doppelseiten-Klemme mit dem Grundkörper 1 aus einem Bauteil besteht.

Am Ausgang 3 des Grundkörpers 1 wird der Adapter 17 befestigt, an dem sich der Urinbeutelanschluss anschließt (Figur 13, 14). Beim Aufstecken des Adapters 17 wird der Schieber 7 auf Stoß in Längsrichtung bewegt, der dadurch in die Öffnungsposition geschoben wird.

Beim Entfernen des Adapter 17 soll das Katheterventil bevorzugt selbsttätig schließen. Dies kann erreicht werden, indem die aufeinander ausgeübte Magnetkraft der Magnete 10, 11 bevorzugt so ausreichend dimensioniert ist, dass die Magnete 10, 11 sich entlang der Ablaufschrägen 8,9 und geführt in dem von den vertikalen Kammern 12b, 13b gebildeten Vertikalführung aufeinander zu bewegen, dabei den längsbeweglichen Schieber 7 zurück in seine Ausgangsstellung drücken und den zwischen den beiden Magneten 10,11 befindlichen Ventilschlauch 6 schließend zusammenquetschen.

### Liste der verwendeten Bezugszeichen

1 - Grundkörper
2 - Eingang
3 - Ausgang
4 - stufenförmige Rasterung
5 - Katheter
6 - Ventilschlauch
7 - längsbeweglicher Schieber
8 - Abschrägung
9 - Abschrägung
10 - Magnet
11 - Magnet
12a - Kammer schräg
13a - Kammer schräg
12b - Kammer vertikal
13b - Kammer vertikal
14 - Nut
15 - Doppelseiten-Klemme
16 - Spitzformgeometrie
17 - Adapter

## Patentansprüche

1. Katheterventil zum Steuern des Fluidflusses eines Mediums, insbesondere ein selbstschliessendes Katheterventil, wobei ein Grundkörper (1) einen Ein-(2) sowie einen Ausgang (3), einen flexiblen Ventilschlauch (6) und als Deckel einen in Längsrichtung beweglichen Schieber (7) sowie am Eingang (2) des Katheterventils eine stufenförmige Rasterung (4) zum Einbinden eines Katheters (5) aufweist und
das Katheterventil im Inneren des zwei seitlich gegenüberliegende vertikale Kammern (12b; 13b) aufweisenden Grundkörpers (1) am längsbeweglichen Schieber (7) zwei gegenläufige Ablaufschrägen (8; 9) besitzt,
***gekennzeichnet dadurch, dass***
der zwei gegenläufige Ablaufschrägen aufweisende Grundkörper (1)
zwei gegenläufige schräge Kammern (12a; 13a) für die Betätigung und Führung von zwei vertikal beweglichen Magneten (10; 11) aufweist, die an zwei gegenüberliegenden Seiten des Ventilschlauches (6) angeordnet sind und dessen Durchfluss wahlweise verschließen oder öffnen, wobei am Ausgang des Katheterventils ein Adapter (17) einzuführen und zu befestigen ist, der - neben dem längsbeweglichen Schieber (7) - zu einer geöffneten Dauerstellung des Katheterventils führt.

2. Katheterventil zum Steuern des Fluidflusses eines Mediums, insbesondere ein selbstschliessendes Katheterventil, wobei ein Grundkörper (1) einen Ein-(2) sowie einen Ausgang (3), einen flexiblen Ventilschlauch (6) und als Deckel einen in Längsrichtung beweglichen Schieber (7) sowie am Eingang (2) des Katheterventils eine stufenförmige Rasterung (4) zum Einbinden eines Katheters (5) aufweist und der Grundkörper (1) in seinem Inneren zwei seitlich gegenüberliegende vertikale Kammern (12b, 13b) besitzt,
***gekennzeichnet dadurch, dass***
der Grundkörper (1) am längsbeweglichen Schieber (7) eine Ablaufschräge (8) sowie eine schräge Kammer (12a) für die Betätigung und Führung von einem vertikal beweglichen Magneten (10) aufweist, der an einer Seite des Ventilschlauches (6) angeordnet ist und dessen Durchfluss wahlweise verschließt oder öffnet, wobei am Ausgang des Katheterventils ein Adapter (17) einzuführen und zu befestigen ist, der - neben dem längsbeweglichen Schieber (7) - zu einer geöffneten Dauerstellung des Katheterventils führt.

3. Katheterventil nach Anspruch 2, ***gekennzeichnet dadurch, dass*** der Grundkörper an seinem Boden eine metallische Platte mit magnetischen Eigenschaften aufweist, gegen die der Magnet anschlägt.

4. Katheterventil nach Anspruch 1 oder 2, ***gekennzeichnet dadurch, dass*** an der stufenförmigen Rasterung (4) des Eingangs (2) des Katheterventils sowie am Katheter (5) eine integrierte oder beidseitig aufsteckbare Sicherung angeordnet ist.

5. Katheterventil nach Anspruch 4, ***gekennzeichnet dadurch, dass*** die Sicherung als U-förmig ausgebildete Doppelseiten-Klemme (15) ausgebildet ist.

6. Katheterventil nach Anspruch 1 oder 2, ***gekennzeichnet dadurch, dass*** der Adapter (17) über einen Überwurfring auf einer Rasterung des Ausgangs des Katheterventils übergreift oder flexibel übergestülpt ist.

7. Katheterventil nach Anspruch 1 oder 2, ***gekennzeichnet dadurch, dass*** ein Entfernen des Adapters (17) aus der Aufnahme des Katheterventils zu einem automatischen Schließen der Magneten (10; 11) und damit des Ventilschlauches (6) führt.

8. Katheterventil nach Anspruch 7, ***gekennzeichnet dadurch, dass*** der Adapter (17) beim Aufstecken den Schieber (7) in Längsrichtungen beweglich ist.

## Claims

1. A catheter valve for controlling the fluid flow of a medium, in particular a self-closing catheter valve, wherein a main body (1) has an inlet (2) as well as an outlet (3), a flexible valve tube (6) and, as a cover, a slider (7) which is movable in a longitudinal direction, as well as a stepped ratchet (4) at the inlet (2) of the catheter valve for the incorporation of a catheter (5), and
inside the main body (1), which has two laterally opposing vertical chambers (12b; 13b), the catheter valve has two counteractive drainage inclines (8; 9) on the longitudinally movable slider (7),
***characterized in that***
the main body (1) having two counteractive drainage inclines has two counteractive inclined chambers (12a; 13a) for actuating and guiding two vertically movable magnets (10; 11) which are disposed on two opposite sides of the valve tube (6) and which selectively close or open the passage thereof, wherein the insertion and attachment of an adapter (17) at the outlet of the catheter valve - in addition to the longitudinally movable slider (7) - will result in a permanently open position of the catheter valve.

2. A catheter valve for controlling the fluid flow of a medium, in particular a self-closing catheter valve, wherein a main body (1) has an inlet (2) as well as an outlet (3), a flexible valve tube (6) and, as a cover, a slider (7) which is movable in a longitudinal direction, as well as a stepped ratchet (4) at the inlet (2) of the catheter valve for the incorporation of a catheter (5), and the main body (1) has inside it two laterally opposing vertical chambers (12b, 13b),
***characterized in that***
the main body (1) has a drainage incline (8) as well as an inclined chamber (12a) on the longitudinally movable slider (7) for the actuation and guidance of a vertically movable magnet (10), which is disposed on one side of the valve tube (6) and selectively closes or opens the passage thereof, wherein the insertion and attachment of an adapter (17) at the outlet of the catheter valve - in addition to the longitudinally movable slider (7) - will result in a permanently open position of the catheter valve.

3. The catheter valve as claimed in claim 2, ***characterized in that*** the main body has a metallic plate with magnetic properties on its bottom, against which the magnet strikes.

4. The catheter valve as claimed in claim 1 or claim 2, ***characterized in that*** a safety device which is integrated or which can be attached to both sides is disposed on the stepped ratchet (4) of the inlet (2) of the catheter valve as well as on the catheter (5).

5. The catheter valve as claimed in claim 4, ***characterized in that*** the safety device is constructed as a U-shaped double-sided clamp (15).

6. The catheter valve as claimed in claim 1 or claim 2, ***characterized in that*** the adapter (17) is engaged over a ratchet of the outlet of the catheter valve, or is flexibly push-fitted over it, via a retaining ring.

7. The catheter valve as claimed in claim 1 or claim 2, ***characterized in that*** a removal of the adapter (17) from the catheter valve receptacle results in an automatic closure of the magnets (10; 11) and therefore of the valve tube (6).

8. The catheter valve as claimed in claim 7, ***characterized in that*** the adapter (17) can be moved in longitudinal directions during attachment of the slider (7).

## Revendications

1. Valve de cathéter destinée à réguler l'écoulement d'un agent, notamment comme valve de cathéter à fermeture automatique, un corps de base (1) présentant une entrée (2) et une sortie (3), un tube flexible (6) et, en guise de couvercle, un curseur (7) déplaçable longitudinalement, ainsi qu'une cannelure en gradins (4) à l'entrée (2) de la valve de cathéter pour la mise en place d'un cathéter (5), et
la valve de cathéter étant munie, à l'intérieur du corps de base (1) qui présente deux chambres verticales (12b; 13b) latéralement opposées, de deux inclinaisons (8; 9) d'orientation opposée dans le curseur (7) déplaçable longitudinalement,
***caractérisée en ce que***
le corps de base (1) qui présente deux inclinaisons d'orientation opposée présente deux chambres obliques (12a; 13a) d'orientation opposée pour actionner et guider deux aimants (10; 11) mobiles verticalement lesquels sont agencés à deux côtés opposés du tube flexible (6) et, au choix, ouvrent ou obturent le passage du tube, un adaptateur (17) devant être introduit dans la sortie de la valve de cathéter et fixé, cet adaptateur conduisant - à côté du curseur (7) déplaçable longitudinalement - à une ouverture permanente de la valve de cathéter.

2. Valve de cathéter destinée à réguler l'écoulement d'un agent, notamment comme valve de cathéter à fermeture automatique, un corps de base (1) présentant une entrée (2) et une sortie (3), un tube flexible (6) et, en guise de couvercle, un curseur (7) déplaçable longitudinalement, ainsi qu'une cannelure en gradins (4) à l'entrée (2) de la valve de cathéter pour la mise en place d'un cathéter (5), et le corps de base (1) ayant dans son intérieur deux chambres verticales (12b; 13b) latéralement opposées,
***caractérisée en ce que***
le corps de base (1) présentant au curseur (7) déplaçable longitudinalement une inclinaison (8) ainsi qu'une chambre oblique (12a) pour actionner et guider un aimant (10) mobile verticalement agencé à un côté du tube flexible (6) dont il ouvre ou obture, au choix, le passage, un adaptateur (17) devant être introduit dans la sortie de la valve de cathéter et fixé, cet adaptateur conduisant - à côté du curseur (7) déplaçable longitudinalement - à une ouverture permanente de la valve de cathéter.

3. Valve de cathéter selon la revendication 2, ***caractérisée en ce que*** le corps de base présente à sa base une plaque métallique avec des propriétés magnétiques contre laquelle l'aimant vient buter.

4. Valve de cathéter selon la revendication 1 ou 2, ***caractérisée en ce qu'***au niveau de la cannelure (4) de l'entrée (2) de la valve de cathéter ainsi qu'au cathéter (5) est agencée une pièce de sécurité intégrée ou pouvant être emboîtée des deux côtés.

5. Valve de cathéter selon la revendication 4, ***caractérisée en ce que*** la pièce de sécurité est réalisée sous forme de pince double (15) en forme de U.

6. Valve de cathéter selon la revendication 1 ou 2, ***caractérisée en ce que*** l'adaptateur (17) peut, par une bague de fixation, recouvrir un crantage de la sortie de la valve de cathéter ou le coiffe de façon flexible.

7. Valve de cathéter selon la revendication 1 ou 2, ***caractérisée en ce que*** le retrait de l'adaptateur (17) du logement de la valve de cathéter entraîne automatiquement la fermeture des aimants (10; 11) et, de ce fait, du tube flexible (6).

8. Valve de cathéter selon la revendication 7, ***caractérisée en ce que*** l'adaptateur (17), lors de sa mise en place, déplace longitudinalement le curseur (7).
